# EUROPEAN PATENT SPECIFICATION

(11) **EP 3 072 532 B1**
(45) Date of publication and mention of the grant of the patent: **09.05.2018**
(21) Application number: 16161409.4
(22) Date of filing: 21.03.2016
(51) Int. Cl.: A61L 2/26, B08B 3/06

(54) **CONTAINING DEVICE FOR A MACHINE FOR WASHING AND/OR STERILIZING LOOSE PRODUCTS**
FASSUNGSBEHÄLTER FÜR EINE MASCHINE ZUM WASCHEN UND / ODER STERILISIEREN LOSE PRODUKTE
ENCIENTE DE CONFINEMENT POUR UNE MACHINE DE LAVAGE ET / OU STÉRILISATION PRODUITS EN VRAC

(30) Priority: 23.03.2015 IT UD20150039
(43) Date of publication of application: 28.09.2016
(73) Proprietor: Icos Pharma S.p.A., 33080 Zoppola (IT)
(72) Inventor: Casonato, Ottorino, 31033 Castelfranco Veneto (TV) (IT); Zardini, Fabio, 31033 Castelfranco Veneto (IT)
(74) Representative: Petraz, Davide Luigi

(56) References cited:
- EP-A1- 0 575 005
- DE-A1- 3 248 555
- US-A1- 2002 056 470

## Description

### FIELD OF THE INVENTION

Embodiments described here concern a containing device for loose products for a machine for treating them, usable, for example, in the pharmaceutical, hospital or food sectors, or in the industrial sector for the production of loose products.

In particular, embodiments described here concern a containing device for loose products which can be, for example, stoppers for test tubes used in laboratory analyses, stoppers for phials of medicine, or small instruments or objects used in operating theatres or laboratories.

### BACKGROUND OF THE INVENTION

It is known, in the pharmaceutical or hospital fields, or in other fields, for example food, that it is necessary to treat, such as for example to wash and/or sterilize, loose products in washing and/or sterilizing machines provided with a sealed treatment chamber, delimited by walls, in which a rotating drum receives the products to be treated on a loading side, washes and/or sterilizes them during a treatment cycle, and then unloads them on an unloading side, where they are generally packaged in sterile packages.

The rotating drums of such machines are partitioned by a multitude of containing devices, such as angular containing compartments for example, typically from four to twenty four, each able to contain, in every operating cycle, a desired quantity of loose products to be treated.

Among the loose products that are normally treated, it is possible to include, for example, stoppers for closing test tubes intended to contain substances to be subjected to laboratory analyses, stoppers for phials of drugs or generally for containers for medicines, or small instruments or objects used in operating rooms or laboratories. These can be made for example of rubber, plastic, polymer materials, metals or metal alloys.

It is known that it is necessary to load loose products manually and/or automatically inside the containing devices to perform the washing and/or sterilizing treatments.

It is also known that it is necessary to unload the loose products from the containing devices after they have been washed and/or sterilized.

It is known that the loading side and the unloading side of such machines are provided with apertures through which to insert, or vice versa to remove, the loose products, respectively to be washed and/or sterilized or after they have been washed and/or sterilized, into/from the angular containing compartments of the rotating drum.

One disadvantage of the state of the art is that the loading and unloading steps of the loose products can be difficult, because the products are loaded and unloaded incoherently, interacting with each other in collisions, knocks, and unpredictable movements and therefore they can come out of the containing devices randomly and uncontrollably, falling both inside and outside the treatment machine, with all the inevitable disadvantages that this entails.

In particular, one disadvantage that is found, during the loading steps, when the loose products exit in an unwanted manner from the containing devices of the machine, is an extension of the time needed for them to be completely loaded and the need for a possible new metering of the loose products.

Another disadvantage, if the loose products come out inside the machine, is that they can possibly accumulate between the rotating drum and the walls that delimit the treatment chamber, leading to interference between the fixed and rotating elements that make up the machine. The presence of free loose products in this zone is unwanted and often not permitted in the health applications in question.

Depending on the frequency and quantity of this accumulation of loose products in sensitive zones of the machine that are not easily reachable, periodic inspections and maintenance must be provided, with consequent stoppage of the machine.

In the worst case scenario, the accumulation of loose products can cause mechanical interferences between the moving and fixed parts, with a consequent blockage of the machine or possibly breakage of the components or mechanisms of the machine.

Document US-A-2002/0056470 describes a known washing and drying machine, in particular for washing and drying impermeable sports equipment.

Document DE-A-3248555 describes a machine for washing small elements, such as stoppers used in the pharmaceutical field.

Document EP-A-0575005 describes an apparatus for washing and sterilizing rubber stoppers or suchlike, usable in pharmaceutical containers.

There is therefore a need to perfect a containing device for loose products that can overcome at least one of the disadvantages of the state of the art.

In particular, one purpose of the present invention is to obtain a containing device that is efficient in preventing loose products from escaping therefrom.

Another purpose of the present invention is to obtain a containing device for loose products that is fast and reliable to load and unload and that facilitates these operations.

Another purpose of the present invention is to obtain a containing device that is simple and precise for loading and unloading loose products.

Another purpose of the present invention is to obtain a containing device that is practical and economical.

The Applicant has devised, tested and embodied the present invention to overcome the shortcomings of the state of the art and to obtain these and other purposes and advantages.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

### SUMMARY OF THE INVENTION

The present invention is set forth and characterized in the independent claims, while the dependent claims describe other characteristics of the invention or variants to the main inventive idea.

In accordance with embodiments described here, a containing device is provided for a machine for washing and/or sterilizing loose products.

According to the present invention, the containing device for loose products comprises:
- a containing body provided internally with a treatment compartment, which has a loading/unloading aperture,
- a closing unit, selectively mobile between an open condition, in which it allows passage through the loading/unloading aperture in order to load and/or unload the loose products into/from the treatment compartment, and a closed condition, in which it prevents passage through the loading/unloading aperture.

According to the present invention, the closing unit of the containing device comprises a door and lateral containing walls that project from lateral edges of the door, configured to contain the loose products laterally, loaded or unloaded in an incoherent form into/from the treatment compartment, when the closing unit is in the open condition.

According to the present invention, the lateral containing walls create a continuity between the door and the containing body, so as to reduce to a minimum or eliminate the possibility of the loose products not reaching the treatment chamber during loading.

According to variant embodiments, containing devices according to the present invention can be part of a treatment machine for washing and/or sterilizing loose products, provided with a treatment chamber inside which a rotating drum is rotatably housed, comprising a plurality of said containing devices.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a three-dimensional view of a containing device in one operating condition according to embodiments described here;
- fig. 2 is a three-dimensional view of a containing device in another operating condition according to embodiments described here;
- fig. 3 is a lateral cross section of the containing device in fig. 2;
- fig. 4 is a three-dimensional view of a containing device according to embodiments described here;
- fig. 5 is a lateral section of a machine for treating loose products in an operating condition with a rotating drum that comprises a plurality of containing devices according to embodiments described here;
- fig. 6 is a lateral section of a machine for treating loose products in another operating condition with a rotating drum that comprises a plurality of containing devices according to embodiments described here;
- fig. 7 is a three-dimensional view of a containing device according to other embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described here concern a containing device 10 for loose products, which can be used in a treatment machine 46, which can be configured to perform a treatment process, or cycle, for example washing and/or sterilizing the loose products, containing them in a plurality of containing devices 10, separated from each other. The treatment machine 46 is generally provided with a treatment chamber 42 inside which the desired treatment cycle is carried out.

Typically, the process conditions can provide specific conditions of heat or heat-moisture, obtained for example by generating or introducing in the treatment chamber 42 heat and/or steam at a desired temperature, chemical conditions, obtained for example by inserting into the treatment chamber 42 a determinate quantity of chemical agents functional for washing and/or sterilizing, pressure conditions, for example by subjecting the treatment chamber 42 to a vacuum.

According to variant embodiments, the treatment of the loose products can also include using a mixture of hot water and silicone, typically in order to protect them.

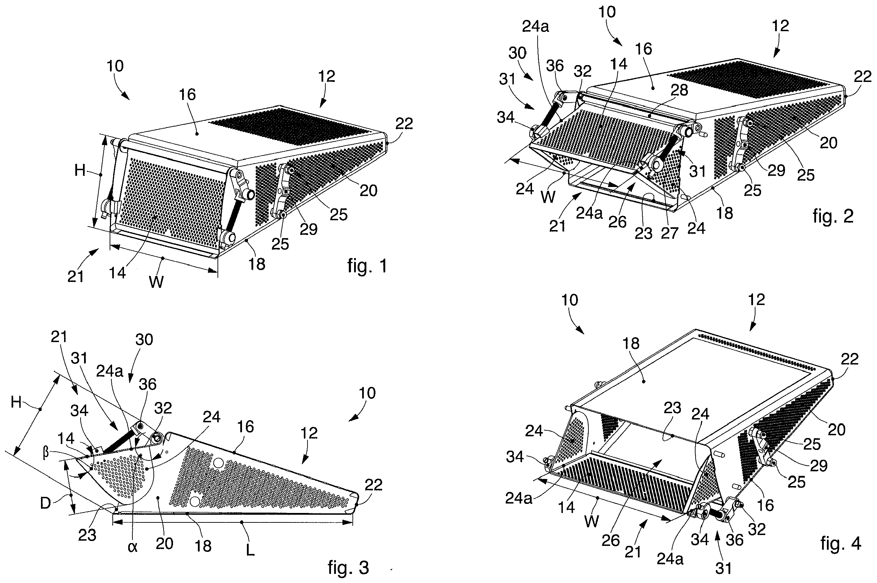

According to some embodiments, described with reference to figs. 1-4, the containing device 10 can comprise a containing body 12, or tub, provided internally with a treatment compartment 26 and which has a loading/unloading aperture 23 which allows to load or unload the loose products in the treatment compartment 26.

The containing device 10 also comprises a closing unit 21, selectively mobile between an open condition, in which it allows passage through the loading/unloading aperture 23 for loading and/or unloading the loose products, and a closed condition, in which it prevents passage through the loading/unloading aperture 23.

According to the present description, the closing unit 21 comprises a closing element, or door 14. Furthermore, according to the present description, the closing unit 21 includes lateral containing walls 24 which project from lateral edges 24a of the door 14 and are configured to laterally contain the loose products loaded or unloaded in incoherent form into/from the treatment compartment 26 when the closing unit 21 is in the open condition.

Typically, the loading/unloading aperture 23 is delimited peripherally by a perimeter edge or frame 27.

The door 14 has a shape and size mating with those of the loading/unloading aperture 23 delimited by the perimeter edge or frame 27, so that, when it is positioned in abutment against the perimeter edge 27, it prevents passage through the loading/unloading aperture 23.

When it is in the closed condition, the containing body 12, together with the door 14, can define for example an internal volume of the treatment compartment 26 which can have a prismatic shape with a triangular, or quadrangular base, pyramidal, truncated pyramid or conical or truncated cone shape. According to some embodiments, described with reference to figs. 1, 2 and 4, the containing body 12 includes an upper wall 16, a lower wall 18, two lateral walls 20 and possibly at least one bottom wall 22. Preferably, providing a bottom wall 22 with a certain height prevents, or reduces to a minimum, the possibility that the loose products might jam and remain imprisoned between the upper wall 16 and the lower wall 18. Alternatively, the bottom wall 22 may not be provided, and the upper wall 16 and lower wall 18 can converge until they meet, closing the treatment compartment 26.

The various walls that make up the containing body 12 can be connected to each other by attachment elements or by welding, or the containing body 12 could also be made as a single body.

Furthermore, the containing body 12 externally delimits the treatment compartment 26 and its volume identifies the maximum metered quantity of loose products that can be contained inside.

According to variant embodiments described using figs. 1, 2 and 4, the containing device 10 is made in the shape of a prism with a quadrangular base, where the bases of the prism correspond to the lateral walls 20, while three of the four lateral faces of the prism correspond respectively to the upper wall 16, the bottom wall 22 and the lower wall 18. The fourth face of the prism corresponds to the door 14, when it is in a closed condition, and is positioned on the opposite side to that represented by the bottom wall 22.

In possible implementations, the shape of the door 14 can be rectangular, for example, having two opposite sides of a size substantially equal to the sides of the perimeter edge 27 that delimits the loading/unloading aperture 23 defined by the upper wall 16 and the lower wall 18, and the other opposite sides with a size substantially equal to the sides of the perimeter edge 27 that delimits the loading/unloading aperture 23 defined by the lateral walls 20.

According to variant embodiments described using figs. 1-4, the first two sides of the rectangle of the door 14 can represent a width W of the door 14, while the second two sides can represent a height H of the door 14.

In possible implementations, the lateral containing walls 24 are connected at the sides or ends of the door 14, and have a size mating with that of the perimeter edge 27 which delimits the loading/unloading aperture 23, that is, essentially equal to height H.

According to variant embodiments described with reference to figs. 2 and 4, the lateral containing walls 24 are transverse to the plane containing the door 14. Moreover, the lateral containing walls 24 can slide internally, parallel and flush with the lateral walls 20, when the door 14 is selectively driven to open or close.

According to other variant embodiments, the lateral containing walls 24 could also slide externally, parallel and flush with the lateral walls 20, when the door 14 is selectively driven to open or close.

By "flush" we mean that the lateral containing walls 24 are at most displaced from the nearest lateral wall 20 by a measurement less than the minimum sizes of the loose products to be contained inside the containing device 10 during the treatment steps. Preferably, the lateral containing walls 24 are not in contact with the lateral walls 20, to prevent wear due to chafing between the two, during the repeated opening and closing steps of the containing device 10.

According to variant embodiments, the lateral containing walls 24 can be triangular, quadrangular or generally polygonal, possibly provided with curved connection parts between the various sides.

In possible embodiments, described with reference to figs. 3 and 4, the lateral containing walls 24 are lobe-shaped, defined by a straight side and a curved convex side.

Advantageously, each lateral containing wall 24 has the size of its lateral edge 24a, that is, the side shared with the door 14, substantially equal to the height H. Furthermore, the two sides adjacent to the lateral edge 24a respectively form two angles, of which one has an amplitude α equal to or less than that of the angle that the door 14 forms, when it is closed, with the upper wall 16, while the other angle has an amplitude β equal to or less than that of the angle that the door 14 forms, when it is closed, with the lower wall 18.

According to variants described using fig. 3, the two sides as above are connected by a curved connection segment, to define the whole shape of the lateral containing wall 24.

The distance D between the tip of the lateral containing wall 24 farthest from the lateral edge 24a is preferably a measurement substantially equal to the height H of the door 14, in order to maximize the containing and guide effect for the loose products during loading and unloading, and is preferably slightly less than the height H of the door 14 so as not to come into contact with the lower wall 18 of the containing body 12 when the door 14 is opening or closing.

In possible implementations, the sizes of the lateral containing walls 24 and the size of the height H of the door 14 can be comprised for example in the range between one third and one fourth of a length L of the lower wall 18.

According to variant embodiments, the lateral containing walls 24 can be connected to the door 14 for example by attachment elements, or by welding.

According to other variants, the lateral containing walls 24 and the door 14 can be obtained by a single mold.

According to other embodiments, the lateral containing walls 24 and the door 14 can be made as a single flat body and subsequently the lateral containing walls 24 are bent with respect to the door 14 to take them to the transverse configuration as described above.

The door 14 can be hinged to the containing body 12, for example by a pin 28, rotatable with respect to the latter to close and keep closed the loading/unloading aperture 23, delimited by the perimeter edge 27 during the washing and/or sterilization treatment, and on the contrary to keep the loading/unloading aperture 23 open during the loading and unloading of the loose products.

During the opening and closing of the door 14, each point of the lateral containing wall 24 is always at a distance from the pin 28 that is less than the perpendicular distance between the pin 28 and the lower wall 18.

In some embodiments, the closing unit 21 can include an opening/closing device 30 connected to the door 14 to move it from the closed position to the open position and vice versa with respect to the loading/unloading aperture 23

In possible variants, described in figs. 1-4, the opening/closing device 30 can be symmetrical with respect to the center line of the containing body 12 and can comprise by way of example two opening/closing units 31 disposed at opposite sides of the perimeter edge 27 delimiting the loading/unloading aperture 23.

The opening/closing units 31 are both connected to the door 14 to make it selectively assume the closed position, for example shown in fig. 1, in which the door 14 covers the opening/closing aperture 23, and the open position, shown in figs. 2 and 4, in which the door 14 is open, freeing the opening/closing aperture 23.

According to the embodiments described using figs. 1-4, the opening/closing units 31 can be connected by a first end 32 to the pin 28 and by a second end 34 to the door 14. In an intermediate position between the first end 32 and the second end 34 an articulation 36 can be made, which allows the selective passage of the door 14 from a closed position to an open position and vice versa, simply driving by thrusting said articulation 36.

According to variants described using figs. 1, 2 and 4, the containing device 10 can be provided with at least one attachment unit 29, firmly anchored by attachment elements 25, for example to the lateral walls 20.

According to variant embodiments, the containing body 12, the door 14 and the lateral containing walls 24 can be holed, allowing possible liquids or mixtures, used during the treatment of the loose products, to enter and exit into and from the containing device 10, so as to prevent any stagnation thereof.

According to variant embodiments, the containing body 12, the door 14 and the lateral containing walls 24 are completely holed on the whole of their surface.

According to other variants, the containing body 12, the door 14 and the lateral containing walls 24 are partly holed on their surface, for example confining the holes to specific areas.

Preferably, no holes are provided on the external profile of the walls that make up the containing body 12, the door 14 and the lateral containing walls 24 so as not to weaken the structure of the containing device 10 excessively.

According to some embodiments, the holes have a diameter less than the sizes of the loose products that they contain, for example between 2 mm and 7 mm, more preferably between 3 mm and 6 mm. The disposition of the holes can be such that each hole is equidistant from its closest neighbors.

Furthermore, all the components of the containing device 10 can be made for example of steel, which is a material suitable for health applications, that is, compatible with entering into contact with the process space of the treatment machine 46.

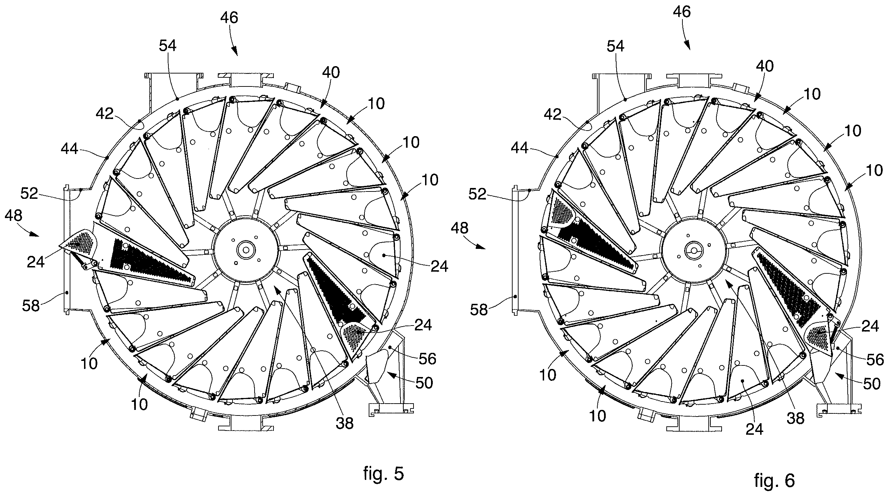

According to the variants described in figs. 5 and 6, a treatment machine 46 contains a plurality of containing devices 10 disposed radially, attached by the attachment units 29 to a frame 38, to define a rotating drum 40.

We must clarify here that for requirements of illustration, in figs. 5 and 6, only some of the containing devices 10 are holed, but advantageously, for the reasons described above, all of them can be.

The treatment machine 46 also comprises a treatment chamber 42 defined by walls 44 that enclose it, defining for example a cylindrical shape inside which the rotating drum 40 is contained.

According to variant embodiments, the treatment machine 46 comprises at least one loading zone 48, to load the loose products into the rotating drum 40, and at least one unloading zone 50, to unload the loose products after washing and/or sterilization, for example positioned on the opposite side of the treatment machine 46 with respect to the loading zone 48.

According to other variant embodiments, it may be provided for example that the loading zone 48 can be situated at the front of the treatment machine 46, and on the contrary that the unloading zone 50 is situated at the rear of the treatment machine 46.

The loading zone 48 can be provided with a manual loading aperture 52 made in the central wall 44 and selectively openable and closable by a loading door 58, to load the loose products into the containing devices 10 of the rotating drum 40.

According to variant embodiments, an automatic loading aperture 54 can be provided, in addition to or as an alternative to the manual loading aperture 52, made in the central wall 44.

According to variant embodiments, an unloading aperture 56 is provided near the unloading zone 50, also made in the central wall 44, to unload the loose products from the containing devices 10 of the rotating drum 40.

According to variants described using fig. 5, when there is a loading of the loose products, one of the containing devices 10 is situated near the manual loading aperture 52 (or the automatic loading aperture 54, not shown) with the door 14 open.

Advantageously, the lateral containing walls 24 create a continuity between the door 14 and the lateral walls 20 and the door 14 creates a continuity with the central wall 44, surrounding the manual loading aperture 52 (or the automatic loading aperture 54, not shown), so as to reduce to a minimum or eliminate the possibility of the loose products not reaching the treatment chamber 26 during loading.

According to variants described using fig. 6, when there is an unloading of the loose products, one of the containing devices 10 is situated near the unloading aperture 56 with the door 14 open.

Advantageously, the lateral containing walls 24 create a continuity between the door 14 and the lateral walls 20 and the door 14 creates a continuity with the central wall 44, surrounding the unloading aperture 56 so as to reduce to a minimum or eliminate the possibility of the loose products not being unloaded from the treatment machine 46 during unloading, for example remaining between the central wall 44 and the rotating drum 40.

The containing device 10 can be made of metal, for example steel, in particular stainless steel,

It is clear that modifications and/or additions of parts may be made to the containing device 10 as described heretofore, without departing from the field and scope of the present invention.

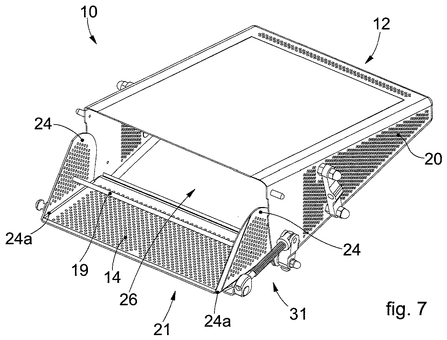

Fig. 7 is used to describe other embodiments, combinable with all the embodiments described here, in which the containing device 10 comprises an oblong stiffening element 19 extending between the two lateral containing walls 24. The oblong stiffening element 19 is connected to the respective internal surfaces of the two lateral containing walls 24. For example, the oblong stiffening element 19 can be a bar, a rod or similar stiffening element. In some embodiments, the oblong stiffening element 19 can be made of metal, for example steel, in particular stainless steel. In possible implementations, the oblong stiffening element 19 can be welded or attached by mechanical attachment elements. The sizes of the oblong stiffening element 19 are such that it does not interfere with the passage of the loose products from and toward the inside of the containing device 10. The oblong stiffening element 19 can be useful to make the structure of the door 14 in general rigid, and in particular, to prevent unwanted bends or deformations of the lateral containing walls 24.

It is also clear that, although the present invention has been described with reference to some specific examples, a person of skill in the art shall certainly be able to achieve many other equivalent forms of containing device 10, having the characteristics as set forth in the claims and hence all coming within the field of protection defined thereby.

Although the above refers to embodiments of the invention, other embodiments can be provided without departing from the main field of protection, which is defined by the following claims.

In the following claims, the sole purpose of the references in brackets is to facilitate reading: they must not be considered as restrictive factors with regard to the field of protection claimed in the specific claims.

## Claims

1. Containing device for a machine for washing and/or sterilizing loose products, comprising:
- a containing body (12) provided internally with a treatment compartment (26), which has a loading/unloading aperture (23),
- a closing unit (21), selectively mobile between an open condition, in which it allows passage through the loading/unloading aperture (23) in order to load/unload the loose products into/from the treatment compartment (26), and a closed condition, in which it prevents passage through the loading/unloading aperture (23),
**characterized in that** said closing unit (21) comprises a door (14) and lateral containing walls (24) that project from lateral edges (24a) of the door (14); configured to laterally contain the loose products, loaded or unloaded in an incoherent form into/from the treatment compartment (26), when the closing unit (21) is in the open condition.

2. Containing device as in claim 1, **characterized in that** said lateral containing walls (24) are transverse to a lying plane of the door (14) and are inside the treatment compartment (26) in the closed condition of the closing unit (21).

3. Containing device as in claim 1, **characterized in that** said lateral containing walls (24) are transverse to a lying plane of the door (14) and are outside the treatment compartment (26) in the closed condition of the closing unit (21).

4. Containing device as in any claim hereinbefore, **characterized in that** each of the lateral containing walls (24) has a lower side in common with a respective lateral edge (24a) of the door (14).

5. Containing device as in claim 4, **characterized in that** the door (14) has a width (W) between the respective two lateral edges (24a) and a height (H) equal to the length of the lateral edges (24a) and a distance (D) between a distal end of each lateral containing wall (24) and the respective lateral edge (24a) of the door (14) is at most equal to the height (H) of the door (14).

6. Containing device as in any claim hereinbefore, **characterized in that** said lateral containing walls (24) have a lobe shape defined by a straight side and a curved convex side.

7. Containing device as in any claim hereinbefore, **characterized in that** said containing body (12) comprises an upper wall (16), a lower wall (18), two lateral walls (20) and a possible bottom wall (22) that delimit said treatment compartment (26).

8. Containing device as in any claim hereinbefore, **characterized in that** the containing body (12), the door (14) and the lateral containing walls (24) are at least partly holed.

9. Containing device as in any claim hereinbefore, **characterized in that** the closing unit (21) comprises an opening/closing device (30) associated to the door (14) and configured to selectively open/close said door (14).

10. Containing device as in any claim hereinbefore, **characterized in that** it comprises an oblong stiffening element (19) extending between the two lateral containing walls (24).

11. Treatment machine for washing and/or sterilizing loose products, said machine (46) being provided with a treatment chamber (42) inside which a rotating drum (40) is rotatably housed, comprising a plurality of containing devices (10) as in any of the claims from 1 to 10.

## Patentansprüche

1. Behältervorrichtung für eine Maschine zum Waschen und/oder Sterilisieren loser Produkte, aufweisend
- einen Behälterkörper (12), der innen mit einer Behandlungskammer (26) versehen ist, die eine Einlade/Auslade-Öffnung (23) hat,
- eine Schließeinheit (21), die selektiv bewegbar ist zwischen einem Offenzustand, in welchem sie einen Durchgang durch die Belade/Entlade-Öffnung (23) hindurch erlaubt, um die losen Produkte in die/aus der Behandlungskammer (26) einzuladen/ausladen, und einen Schließ-Zustand, in welchem sie einen Durchgang durch die Belade/Entlade-Öffnung (23) hindurch unterbindet,
**dadurch gekennzeichnet, dass** die Schließeinheit (21) eine Tür (14) und seitliche Behälterwände (24) aufweist, die von Seitenrändern (24a) der Tür (14) aus vorstehen und die konfiguriert sind, um die losen Produkte, die in einer unzusammenhängenden Weise in die/aus der Behandlungskammer (26) eingeladen oder ausgeladen werden, seitlich zu fassen, wenn die Schließeinheit (21) im Offenzustand ist.

2. Behältervorrichtung gemäß Anspruch 1, **dadurch gekennzeichnet, dass** die seitlichen Behälterwände (24) quer zu einer Lageebene der Tür (14) sind und innerhalb der Behandlungskammer (26) sind im Schließzustand der Schließeinheit (21).

3. Behältervorrichtung gemäß Anspruch 1, **dadurch gekennzeichnet, dass** die seitlichen Behälterwände (24) quer zu einer Lageebene der Tür (14) sind und außerhalb der Behandlungskammer (26) sind im Schließzustand der Schließeinheit (21).

4. Behältervorrichtung gemäß irgendeinem vorigen Anspruch, **dadurch gekennzeichnet, dass** jede der seitlichen Behälterwände (24) eine untere Seite gemeinsam mit einem jeweils zugeordneten Seitenrand (24a) der Tür (14) hat.

5. Behältervorrichtung gemäß Anspruch 4, **dadurch gekennzeichnet, dass** die Tür (14) eine Breite (W) zwischen den jeweiligen zwei Seitenrändern (24a) und eine Höhe (H) gleich der Länge der Seitenränder (24a) hat, und ein Abstand (D) zwischen einem distalen Ende jeder seitlichen Behälterwand (24) und dem jeweils zugeordneten Seitenrand (24a) der Tür (14) maximal gleich der Höhe (H) der Tür (14) ist.

6. Behältervorrichtung gemäß irgendeinem vorigen Anspruch, **dadurch gekennzeichnet, dass** die seitlichen Behälterwände (24) eine Buckelform haben, die von einer geraden Seiten und einer gekrümmten konvexen Seite definiert wird.

7. Behältervorrichtung gemäß irgendeinem vorigen Anspruch, **dadurch gekennzeichnet, dass** der Behälterkörper (12) eine obere Wand (16), eine untere Wand (18), zwei seitliche Wände (20) und eine mögliche untere Wand (22) aufweist, welche die Behandlungskammer (26) begrenzen.

8. Behältervorrichtung gemäß irgendeinem vorigen Anspruch, **dadurch gekennzeichnet, dass** der Behälterkörper (12), die Tür (14) und die seitlichen Behälterwände (24) wenigstens teilweise gelocht sind.

9. Behältervorrichtung gemäß irgendeinem vorigen Anspruch, **dadurch gekennzeichnet, dass** die Schließeinheit (21) eine Öffnen/Schließen-Vorrichtung (30) aufweist, die mit der Tür (14) in Verbindung steht und die konfiguriert ist, um die Tür (14) selektiv zu öffnen/zu schließen.

10. Behältervorrichtung gemäß irgendeinem vorigen Anspruch, **dadurch gekennzeichnet, dass** sie aufweist ein längliches Versteifungselement (19), das sich zwischen den beiden seitlichen Behälterwänden (24) erstreckt.

11. Behandlungsmaschine zum Waschen und/oder Sterilisieren loser Produkte, wobei die Maschine (46) mit einem Behandlungsraum (42) ausgestattet ist, innerhalb dessen eine rotierende Trommel (40) drehbar untergebracht ist, welche eine Mehrzahl von Behältervorrichtungen (10) gemäß irgendeinem der Ansprüche 1 bis 10 aufweist.

## Revendications

1. Dispositif contenant pour une machine pour le lavage et/ou la stérilisation de produits en vrac, comprenant :
- un corps contenant (12) pourvu à son intérieur d'un compartiment de traitement (26), possédant une ouverture de chargement/déchargement (23),
- une unité de fermeture (21) mobile sélectivement entre une condition d'ouverture, dans laquelle elle permet le passage à travers l'ouverture de chargement/déchargement (23) afin de charger/décharger les produits en vrac dans le/du compartiment de traitement (26), et une condition de fermeture, dans laquelle elle empêche le passage à travers l'ouverture de chargement/déchargement (23),
**caractérisé en ce que** ladite unité de fermeture (21) comprend une porte (14) et des parois de confinement latérales (24), faisant saillie de bords latéraux (24a) de la porte (14) ; conçues pour confiner latéralement les produits en vrac, chargés ou déchargés en forme incohérente dans le /du compartiment de traitement (26), lorsque l'unité de fermeture (21) étant la condition d'ouverture.

2. Dispositif contenant selon la revendication 1, **caractérisé en ce que** lesdites parois de confinement latérales (24) sont transversales par rapport à un plan couche de la porte (14) et se trouvent à l'intérieur du compartiment de traitement (26) dans la condition de fermeture de l'unité de fermeture (21).

3. Dispositif contenant selon la revendication 1, **caractérisé en ce que** lesdites parois de confinement latérales (24) sont transversales par rapport à un plan couche de la porte (14) et se trouvent à l'extérieur du compartiment de traitement (26) dans la condition de fermeture de l'unité de fermeture (21).

4. Dispositif contenant selon n'importe laquelle des revendications précédentes, **caractérisé en ce que** chacune des parois de confinement latérales (24) possède un côté inférieur en commun avec un bord latéral respectif (24a) de la porte (14).

5. Dispositif contenant selon la revendication 4, **caractérisé en ce que** la porte (14) possède une largeur (W) entre les deux bords latéraux respectifs (24a) et une hauteur (H) égale à la longueur des bords latéraux (24a) et une distance (D) entre une extrémité distale de chaque paroi de confinement latérale (24) et le bord latéral respectif (24a) de la porte (14) est au plus égal à la hauteur (H) de la porte (14).

6. Dispositif contenant selon n'importe laquelle des revendications précédentes, **caractérisé en ce que** lesdites parois de confinement latérales (24) ont une forme en lobe définie par un côté rectiligne et un côté convexe courbe.

7. Dispositif contenant selon n'importe laquelle des revendications précédentes, **caractérisé en ce que** ledit corps contenant (12) comprend une paroi supérieure (16), une paroi inférieure (18), deux parois latérales (20) et une possible paroi de fond (22), délimitant ledit compartiment de traitement (26).

8. Dispositif contenant selon n'importe laquelle des revendications précédentes, **caractérisé en ce que** le corps contenant (12), la porte (14) et les parois de confinement latérales (24) sont au moins en partie perforés.

9. Dispositif contenant selon n'importe laquelle des revendications précédentes, **caractérisé en ce que** l'unité de fermeture (21) comprend un dispositif d'ouverture/fermeture (30) associé à la porte (14) est conçu pour ouvrir/fermer sélectivement ladite porte (14).

10. Dispositif contenant selon n'importe laquelle des revendications précédentes, **caractérisé en ce qu'il** comprend un élément raidisseur oblong (19) s'étendant entre les deux parois de confinement latérales (24).

11. Machine de traitement pour le lavage et/ou la stérilisation de produits en vrac, ladite machine (46) étant pourvue d'une chambre de traitement (42) à l'intérieur de laquelle un tambour rotatif (40) est monté tournant, comprenant une pluralité de dispositifs contenants (10) selon n'importe laquelle des revendications 1 à 10.
